# EUROPÄISCHE PATENTSCHRIFT

(11) **EP 1 919 851 B1**
(45) Veröffentlichungstag und Bekanntmachung des Hinweises auf die Patenterteilung: **14.07.2010**
(21) Anmeldenummer: 06778244.1
(22) Anmeldetag: 15.08.2006
(51) Int. Cl.: C07C 29/78, C07C 29/17, C07C 29/56, C07C 35/17, C07C 35/12, B01D 9/00, C11B 9/00

(54) **VERFAHREN ZUR HERSTELLUNG VON ANGEREICHERTEM ISOPULEGOL**
METHOD FOR THE PRODUCTION OF ENRICHED ISOPULEGOL
PROCEDE POUR PREPARER DE L'ISOPULEGOL ENRICHI

(30) Priorität: 26.08.2005 DE 102005040655
(43) Veröffentlichungstag der Anmeldung: 14.05.2008
(73) Patentinhaber: BASF SE, 67056 Ludwigshafen (DE)
(72) Erfinder: RAULS, Matthias, 67061 Ludwigshafen (DE); JÄKEL, Christoph, 67117 Limburgerhof (DE); KASHANI-SHIRAZI, Nawid, 68161 Mannheim (DE); EBEL, Klaus, 68623 Lampertheim (DE)
(86) Internationale Anmeldenummer: PCT/EP2006/065322
(87) Internationale Veröffentlichungsnummer: WO 2007/023109

(56) Entgegenhaltungen:
- EP-A- 1 053 974
- US-A- 3 218 361
- WYNN N P: "SEPARATE ORGANICS BY MELT CRYSTALLIZATION" CHEMICAL ENGINEERING PROGRESS, AMERICAN INSTITUTE OF CHEMICAL ENGINEERS, NEW YORK, NY, US, Bd. 88, Nr. 3, 1. März 1992 (1992-03-01), Seiten 52-60, XP000246045 ISSN: 0360-7275

## Beschreibung

### Technisches Gebiet der Erfindung:

Die vorliegende Erfindung betrifft ein Verfahren zur Herstellung von angereichertem Isopulegol durch Kristallisation aus einer Isopulegol enthaltenden Schmelze. Speziell betrifft die Erfindung ein Verfahren zur Herstellung von enantiomerenangereichertem n-Isopulegol ausgehend von optisch aktivem Isopulegol mit geringerem Enantiomerenüberschuss durch Kristallisation aus der Schmelze. Die Erfindung betrifft darüber hinaus ein Verfahren zur Herstellung von Menthol ausgehend von durch Kristallisation aus der Schmelze hergestelltem enantio- und/oder diastereomerenangereichertem n-Isopulegol.

Menthol ist ein natürlich vorkommender Wirkstoff, der in Pharmazie, Kosmetik und Lebensmittelindustrie breite Anwendung findet. In natürlichen Quellen, beispielsweise dem Pfefferminzöl, kommt Menthol in Form von vier diastereomeren Enantiomerenpaaren vor, von denen nur die Hauptkomponente, das (-)-Menthol die gewünschten geschmacklichen und sonstigen sensorischen Eigenschaften hat. Eine mehrstufige Reinigung natürlichen Menthols ist daher immer unumgänglich. Erzeugt man Menthol synthetisch, so muss durch die Syntheseroute sichergestellt werden, dass das Endprodukt reines (-)-Menthol ist. Da dies im allgemeinen nicht oder nur unvollständig gelingt, müssen auch hier die Reaktionsprodukte mit verfahrenstechnischen Methoden voneinander getrennt werden. Die neben dem (-)- und (+)-Menthol vorliegenden weiteren Diastereomere lassen sich, wenn auch mit erheblichem Aufwand, durch Destillation abtrennen. (-) und (+)-Menthol hingegen können wegen ihrer identischen physikalischen Eigenschaften so nicht getrennt werden.

Verfahren zur Trennung von optischen Isomeren im allgemeinen bzw. (+)- und (-)-Menthol im speziellen sind in großer Zahl bekannt. Die wesentlichen Methodengruppen dabei sind: a) Direkte Kristallisation des Wirkstoffs aus einem Lösemittel oder aus der Schmelze, b) Bildung eines diastereomeren Derivats mit einem chiralen Agens (z.B. in Form eines Salzes oder Esters) und Kristallisation oder Destillation des gebildeten, sich in seinen physikalischen Eigenschaften nun unterscheidenden Diastereomerengemisches, c) chromatographische Methoden und d) enzymatische Methoden. Daneben existieren eher als experimentell zu bezeichnende Labormethoden, die für eine technische Anwendung allerdings keine Rolle spielen. Eine ausführliche Darstellung aller relevanten Methoden und ihrer verschiedenen Ausführungsformen findet sich beispielsweise in Jaques, J. et al., Enantiomers, Racemates and Resolutions, Krieger Publishing Co. 1994.

Die äußerst schwierige direkte Kristallbildung des Menthols ist vielfach untersucht worden. Seit langem bekannt ist, dass Menthol in mehreren Modifikationen kristallisiert, die sich in Schmelzpunktnähe ineinander umwandeln können (siehe K. Schaum, Lieb. Ann. Chem. 308 (1899), S.37). Die beschriebenen Modifikationen bilden, abhängig von der Temperatur, ein äußerst komplexes Phasensystem mit isotrimorpher Mischkristallbildung, wie von Kuhnert-Brandstätter et al. in Archiv der Pharmazie 307 (1974) S. 497 beschrieben. Dieses ungewöhnliche Verhalten führt zu einer weitgehenden gegenseitigen Mischbarkeit der Isomere im Festkörper, sogenannter fester Lösungen, die eine Trennung der Isomere durch direkte Kristallisation meist verhindert.

Stand der Technik:

Eine lösemittelfreie Kristallisation von Menthol aus der Schmelze bei sehr tiefen Temperaturen (-60°C) wird in der WO 03/083028 beschrieben. Dabei wird Menthol aus einem aus natürlichen Quellen gewonnenen, eine Vielzahl weiterer Komponenten enthaltenden Öl isoliert.

Ein vergleichbares Verfahren zur Isolierung von Menthol aus einem essentiellen Öl durch Kristallisation bei Temperaturen bis zu -35°C beschreiben S. Tandon et al. in Journal of Medicinal and Aromatic Plant Sciences, 20, (1998) 25-27.

Die DE 195 36 827-A betrifft ein Verfahren zur Trennung flüssiger eutektischer Mischungen durch Kristallisation an Kühlflächen, auf die Impfkristalle aufgebracht werden, auf denen arteigene Kristalle aufwachsen, welche nach Erwärmung der Flächen in flüssiger Form abgezogen werden.

Synthetisches Menthol wird zumeist über die Zwischenstufe des Isopulegols gewonnen, dass sich vom Menthol nur durch eine Doppelbindung in der iso-Propyl-Seitenkette unterscheidet. Menthol wird hieraus ohne Verlust der Stereospezifität durch Hydrierung freigesetzt. Ein Verfahren, die nur schwer erreichbare Isomerentrennung des Menthols durch Kristallisation zu bewerkstelligen, wird in der FR 1.374.732 offenbart: Dort wird die Reinigung des Isopulegols durch fraktionierte Kristallisation aus Petrolether bzw. Aceton bei Temperaturen zwischen -40°C und -75°C beschrieben. Dadurch wird eine Trennung des Isopulegols von seinen drei weiteren Diastereomeren erreicht.

Die US 5,663,460 offenbart die Aufreinigung von (-)-n-Isopulegol durch Kristallisation aus Petrolether oder vorteilhaft aus Aceton bei Temperaturen von -20°C bis -60°C. Dabei kann auch eine Steigerung der optischen Reinheit erreicht werden. Ein weiteres Verfahren zur Aufreinigung von Isopulegol durch Kristallisation ist in der US 3,218,361 beschrieben.

### Aufgabe der Erfindung:

Ausgehend vom Stand der Technik stellte sich nun die Aufgabe der Bereitstellung eines Verfahrens, das es erlaubt, Isopulegol in hoher chemischer und optischer Reinheit bereitzustellen und sich für eine wirtschaftliche Anwendung im technischen Maßstab eignet. Im Hinblick auf die Anwendung des herzustellenden Isopulegols bzw. dessen Folgeprodukte am Menschen, soll dabei weitgehend auf physiologisch bedenkliche Reagenzien bzw. organische Lösemittel verzichtet werden können.

### Beschreibung der Erfindung sowie der bevorzugten Ausführungsformen:

Die Aufgabe wurde erfindungsgemäß gelöst durch die Bereitstellung eines Verfahrens gemäß Anspruch 1, zur Herstellung von angereichertem Isopulegol der Formel (I) 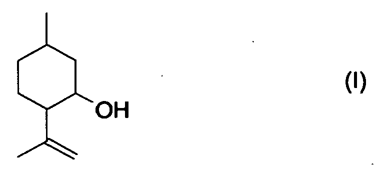 durch Kristallisation aus einer Isopulegol der Formel (I) enthaltenden Schmelze.

Die vorliegende Erfindung betrifft demnach ein Verfahren zur Reinigung von Isopulegol durch Kristallisation aus einer Schmelze, die neben Isopulegol noch andere unerwünschte Verunreinigungen oder Verbindungen, beispielsweise Nebenprodukte, die bei der Herstellung des eingesetzten Isopulegols angefallen sind, enthält, jedoch im wesentlichen frei von Lösungsmitteln ist.

Unter dem Begriff angereichertes Isopulegol ist im Rahmen der vorliegenden Erfindung ein solches zu verstehen, das eine chemische Reinheit von mindestens etwa 90 Gew.-%, bevorzugt mindestens etwa 95 Gew.-% und besonders bevorzugt etwa 95 bis etwa 99,95 Gew.-% aufweist. Dabei ist unter Isopulegol der Formel (I) ein Gemisch der vier möglichen Diastereomere des Isopulegols, nämlich n-Isopulegol, Iso-Isopulegol, Neo-Isopulegol und Neoiso-Isopulegol zu verstehen.

Als Ausgangsstoff zur Durchführung des erfindungsgemäßen Verfahrens eignet sich Isopulegol jeglichen Ursprungs, d.h. aus natürlichen Quellen isoliertes Isopulegol oder synthetisch hergestelltes Isopulegol. Die erfindungsgemäß einzusetzende Schmelze besteht bevorzugt zu mindestens etwa 70 Gew.-%, besonders bevorzugt zu mindestens etwa 75 Gew.-%, ganz besonders bevorzugt zu etwa 80 bis etwa 100 Gew.-% und insbesondere bevorzugt zu etwa 85 bis etwa 100 Gew.-% aus Isopulegol.

Die Erfindung betrifft speziell ein Verfahren zur Herstellung von enantio- und/oder diastereomerenangereichertem n-Isopulegol der Formel (II) 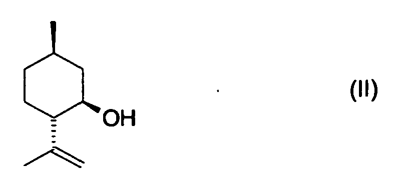 durch Kristallisation aus einer n-Isopulegol der Formel (II) und gegebenenfalls weitere Diastereomere des Isopulegols enthaltenden Schmelze.

Bevorzugte Ausgangsstoffe bzw. deren Schmelzen bestehen zu mindestens etwa 70 Gew.-%, bevorzugt bevorzugt zu mindestens etwa 75 Gew.-% und besonders bevorzugt zu etwa 80 bis etwa 100 Gew.-% und ganz besonders bevorzugt von etwa 85 bis etwa 100 Gew.-% aus n-Isopulegol der Formel (II). Derartige Schmelzen können noch die vorstehend genannten Diastereomere des Isopulegols in veränderlichen Anteilen sowie in veränderlichem Umfang, je nach Ursprung bzw. Art der Herstellung des erfindungsgemäß umzusetzenden n-Isopulegols, enthalten.

Das durch die erfindungsgemäße Kristallisation aus der Schmelze erhältliche n-Isopulegol der Formel (II) fällt üblicherweise in diastereomerenangereicherter Form an. Unter dem Begriff diastereomerenangereichert ist dabei zu verstehen, dass die erfindungsgemäß erhältlichen Produkte einen höheren Gehalt des gewünschten Diastereomeren n-Isopulegol relativ zu den übrigen, vorstehend genannten Diastereomeren aufweisen, als die erfindungsgemäß eingesetzte Schmelze.

Bei Einsatz optisch aktiver Ausgangsstoffe, d.h. Ausgangsstoffen, bei denen die beiden Enantiomere des n-Isopulegols nicht im gleichen Verhältnis vorliegen, erhält man im Rahmen des erfindungsgemäßen Kristallisationsverfahrens enantiomerenangereichertes n-Isopulegol. Unter dem Begriff enantiomerenangereichert ist dabei zu verstehen, dass die erfindungsgemäß erhältlichen Produkte einen höheren Gehalt eines Enantiomeren des n-Isopulegols relativ zum anderen Enantiomeren, d.h. einen höheren Enantiomerenüberschuss (ee) aufweisen, als die erfindungsgemäß eingesetzte Schmelze.

Das erfindungsgemäße Verfahren ermöglicht demnach auch die Herstellung von enantio- und diastereomerenangereichertem n-Isopulegol durch Kristallisation aus einer optisch aktives n-Isopulegol mit geringerem Enantiomerenüberschuss enthaltenden Schmelze.

Erfindungsgemäß bevorzugte Ausgangsstoffe bzw. deren Schmelzen enthalten n-Isopulegol mit einem Enantiomerenüberschuss von mindestens etwa 75 % ee, besonders bevorzugt von mindestens etwa 80 % ee und ganz besonders bevorzugt von etwa 85 bis etwa 90 % ee.

Bei Einsatz wie vorstehend beschriebener optisch aktiver Ausgangsstoffe erhält man im Rahmen des erfindungsgemäßen Verfahrens üblicherweise n-Isopulegol mit einem Enatiomerenüberschuss von mindestens etwa 85% ee, bevorzugt etwa 90 bis etwa 100% ee, besonders bevorzugt etwa 95 bis etwa 99,9% ee und ganz besonders bevorzugt etwa 97 bis etwa 99,9% ee.

Das erfindungsgemäße Kristallisationsverfahren eignet sich im Rahmen einer bevorzugten Ausführungsform zur Herstellung von enantiomerenangereichertem L-(-)-n-Isopulegol der Formel (II*) 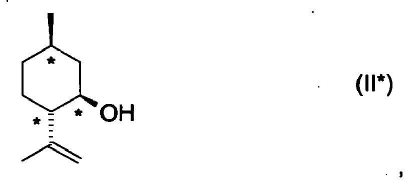 wobei * jeweils ein asymmetrisches Kohlenstoffatom in der dargestellten absoluten Konfiguration bezeichnet, durch Kristallisation von L-(-)-n-Isopulegol enthaltenden Schmelzen.

Der Begriff der Kristallisation aus der Schmelze bzw. Schmelzekristallisation ist dem Fachmann bekannt und ausführlich beispielsweise in G.F. Arkenbout, Melt Crystallization Technology, Lancater/PA, Technomic Publ. Co., 1995 beschrieben. Im Rahmen der vorliegenden Erfindung ist dabei eine Kristallisation zu verstehen, die aus der Schmelze, d.h. aus einem Gemisch aus flüssigem, d.h. geschmolzenem und gegebenenfalls bereits verfestigtem Ausgangsmaterial ohne Zusatz weiterer Komponenten, wie beispielsweise Lösungsmitteln oder anderen Hilfsstoffen, durchgeführt wird.

Die erfindungsgemäße Schmelzekristallisation wird in Form einer Schichtkristallisation durchgeführt. Zur Durchführung einer Schichtkristallisation bringt man üblicherweise eine gekühlte Fläche in die Schmelze des als Ausgangsstoff eingesetzten gegebenenfalls optisch aktiven Isopulegols ein. Daraufhin bildet sich auf der eingebrachten, gekühlten Oberfläche eine Kristallschicht aus gegebenenfalls enantiomeren- und/oder diastereomerenangereichertem Isopulegol, die anschließend von der verbleibenden Mutterschmelze getrennt werden kann. Das so gewonnene kristalline angereicherte Isopulegol kann in weiteren, hilfsstofffreien Reinigungsschritten (z.B. durch Waschen mit Reinprodukt, "Schwitzen" knapp unter dem Schmelzpunkt) wiederum aufgeschmolzen werden. Anschließend kann diese Operation zu Erhöhung der Reinheit und der Ausbeute am aufgeschmolzenen Kristallisat und an der Mutterschmelze beliebig oft wiederholt werden. Generell sind im Rahmen der erfindungsgemäß vorteilhaft durchzuführenden Verfahren der Schichtkristallisation die dynamischen von den statischen Verfahren zu unterscheiden. Bei den dynamischen Verfahren wird die Mutterphase, d.h. der geschmolzene Ausgangsstoff aktiv oder passiv am Kristallisat bzw. der Kühlfläche entlang bewegt. Im Rahmen der statischen Verfahren führt man die erfindungsgemäße Schmelzekristallisation in einer ruhenden Schmelze durch.

Das erfindungsgemäße Verfahren lässt sich demnach auch in Form einer dynamischen Schichtkristallisation durchführen. Im Rahmen einer bevorzugten Ausführungsform führt man diese Variante in Rohrbündelwärmetauschern durch, wie sie in G.F. Arkenbout, Melt Crystallization Technology, Lancater/PA, Technomic Publ. Co., 1995 (Kap. 6.2) beschrieben sind. Dabei werden Schmelze und Kältemittel z.B. in Form eines Rieselfilms an den Innen- und Außenwänden der Wärmetauscher entlanggeführt. Eine derartige Vorrichtung erlaubt eine erleichterte Abtrennung des erhaltenen kristallinen Isopulegols von der Mutterschmelze bzw. gegebenenfalls erhaltenen Schwitzfraktionen durch einfaches Ablaufen unter Schwerkrafteinfluss und bedarf, außer einer Umwälzpumpe, keiner weiteren Rührorgane.

Zur Durchführung einer dynamischen Schichtkristallisation füllt man das als Ausgangssubstanz dienende, gegebenenfalls optisch aktive Isopulegol üblicherweise mit einer aus dem Schmelzdiagramm ablesbaren Temperatur oberhalb seines Schmelzpunktes in den wie zuvor beschriebenen Schmelzekristallisator ein und führt es durch Umpumpen durch den gekühlten Rohrbündelwärmetauscher. Zur Erzielung eines vorteilhaften Kristallisationsergebnisses wird die Absenkung der Kälteträgertemperatur vorzugsweise so gewählt, dass sich innerhalb eines Zeitraumes von etwa 0,5 h bis etwa 10 h, bevorzugt innerhalb von etwa 1 h bis etwa 4 h eine Kristallschicht einer Stärke von etwa 1 mm bis etwa 50 mm, bevorzugt etwa 5 mm bis etwa 20 mm bildet. Die hierfür erforderlichen Kältemitteltemperaturen liegen in der Regel um etwa 1 K bis etwa 40 K, bevorzugt um etwa 5 K bis etwa 20 K unterhalb der jeweiligen Schmelzetemperatur.

Nach Durchführung der dynamischen Schichtkristallisation wird die verbleibende Mutterschmelze üblicherweise abgelassen. Durch Anheben der Temperatur des Heiz-bzw. Kühlmediums des Wärmetauschers können gegebenenfalls anhaftende Mutterschmelzenreste bzw. gegebenenfalls eingeschlossene Verunreinigungen aufgeschmolzen bzw. durch Drainage entfernt. Vorteilhafte Wärmeträgertemperaturen liegen im Bereich von etwa 15°C bis etwa 60°C, besonders vorteilhaft von etwa 20 bis etwa 30°C. Bei diesem als "Schwitzen" zu bezeichnenden Prozess können je nach Reinheitsanforderungen etwa 1 bis etwa 50 Gew.-%, oft etwa 5 bis etwa 20 Gew.-% des kristallisierten Isopulegols wieder aufgeschmolzen werden. Abschließend wird die verbleibende enantio- bzw. diastereomerenangereicherte Kristallschicht vorteilhaft abgeschmolzen und entweder ihrer weiteren Verwendung zugeführt oder nochmals zur weiteren Aufreinigung bzw. Steigerung des Enatiomeren- bzw. Diastereomerenüberschusses kristallisiert. Die wie beschrieben abgetrennte Mutterschmelze und die durch Schwitzen freigesetzte Fraktion können zur Erhöhung der Ausbeute in das erfindungsgemäße Verfahren zurückgeführt werden. Alternativ besteht die Möglichkeit, vor dem "Schwitzen" der Kristallschicht diese durch Inkontaktbringen mit aufgeschmolzenem Reinprodukt zu waschen, also von gegebenenfalls fest anhaftender Mutterlauge zu befreien.

Die erfindungsgemäße Schmelzekristallisation kann alternativ dazu auch in Form einer Suspensionskristallisation durchgeführt werden. Dabei werden die Kristalle üblicherweise in suspendierter Form in ihrer Mutterschmelze erzeugt, ohne dass sich eine Kristallschicht bilden muss. Hierbei sind eine kontinuierliche Fahrweise bei konstanter Temperatur und eine diskontinuierliche Fahrweise mit sukzessive abgesenkter Temperatur möglich. Als Kühlfläche kommen hier beispielsweise mit einem wandgängigen Rührer ausgestatteten Wände eines Rührbehälters in Frage, sogenannte Kratzkühler oder die gewischten Flächen in einem Kühlscheibenkristallisator. Alternativ kann auch durch Anlegen eines Vakuums und adiabatische Verdampfung des Wertstoffs (bzw. weniger bevorzugt, eines hilfsweise zugegebenen Lösemittels) die Schmelze abgekühlt werden. Die Abtrennung der suspendierten Kristalle kann dann auf dem Fachmann an sich bekannte Weise, z.B. mit einem beliebigen Filterorgan erfolgen, z.B. einer Nutsche, einer Zentrifuge oder einem Bandfilter. Wegen der prinzipiell erreichbaren, extrem hohen Reinigungswirkung kann die Abtrennung auch mittels einer Waschkolonne erfolgen, wobei der von oben an ein Filter herangeführten Suspension von unten aufgeschmolzenes Reinprodukt als Waschmittel entgegengeführt wird.

Die erfindungsgemäße Kristallisation von Isopulegol bzw n-Isopulegol aus der Schmelze wird vorteilhaft bei Temperaturen im Bereich von etwa -20°C bis etwa 15°C, bevorzugt im Bereich von etwa -10°C bis etwa 15°C und besonders bevorzugt im Bereich von etwa -5°C bis etwa 14°C durchgeführt. Die genaue Lage des Temperaturbereiches hängt allein von der optischen und chemischen Eingangsreinheit des Ausgangsmaterials und der gewünschten Ausbeute ab und kann vom Fachmann aus dem Schmelzdiagramm des jeweils eingesetzten Isopulegols abgelesen werden.

Im Falle des erfindungsgemäßen Herstellverfahrens für angereichertes, bevorzugt enantiomeren- bzw. diastereomerenangereichertes Isopulegol können alle genannten Methoden mit gutem Erfolg eingesetzt werden. Im Rahmen einer bevorzugten Ausführungsform des erfindungsgemäßen Verfahrens führt man die Kristallisation in einem statischen Schichtkristallisator mit internen Wärmetauscherflächen durch. An die Anordnung der genannten Wärmetauscherflächen sind keine besonderen Anforderungen zu stellen. Üblicherweise füllt man das als Ausgangssubstanz dienende Isopulegol mit einer aus dem Schmelzdiagramm ablesbaren Temperatur oberhalb seines Schmelzpunktes in den Schmelzekristallisator ein und kühlt den Inhalt des Kristallisators innerhalb eines Zeitraums von etwa 5 h bis etwa 30 h, bevorzugt von etwa 10 bis etwa 20 h abhängig von der Reinheit des Ausgangsmaterials auf Temperaturen von etwa -20°C bis etwa 15°C, bevorzugt von etwa -10°C bis etwa 15°C ab. Zur Erzielung eines vorteilhaften Kristallisationsergebnisses werden dabei bevorzugt Abkühlgeschwindigkeiten von etwa 0,1 K/h bis etwa 20 K/h, besonders bevorzugt von etwa 0,5 K/h bis etwa 5 K/h gewählt.

Nach Kristallisation der gewünschten Menge Ausagangsmaterials wird die verbleibende Mutterschmelze vorteilhaft abgelassen. Durch langsames Anheben der Temperatur des Heiz-/Kühlmediums des Wärmetauschers können gegebenenfalls anhaftende Mutterschmelzenreste bzw. gegebenenfalls eingeschlossene Verunreinigungen aufgeschmolzen bzw. durch Drainage entfernt werden. Vorteilhafte Aufheizgeschwindigkeiten liegen im Bereich zwischen etwa 0,1 und etwa 20 K/h, bevorzugt im Bereich von etwa 0,5 bis etwa 5 K/h. Bei diesem als "Schwitzen" zu bezeichnenden Prozess können je nach Reinheitsanforderungen etwa 3 bis etwa 60 Gew.-%, oft etwa 10 bis etwa 30 Gew.-% des kristallisierten Isopulegols wieder aufgeschmolzen werden. Abschlieβend können die verbleibende enantiomerenangereicherte Kristallschicht vorteilhaft abgeschmolzen und entweder ihrer weiteren Verwendung zugeführt oder nochmals zur weiteren Aufreinigung bzw. Steigerung des Enatiomerenüberschusses kristallisiert werden. Die wie beschrieben abgetrennte Mutterschmelze und die durch Schwitzen freigesetzte Fraktion können zur Erhöhung der Ausbeute in das erfindungsgemäße Verfahren zurückgeführt werden.

Das erfindungsgemäße Verfahren eröffnet einen wirtschaftlich besonders vorteilhaften Zugang zu enantiomeren- bzw. diastereomerenangereichertem Isopulegol ausgehend von Isopulegol mit geringerer Enantiomeren- bzw Diastereomerenreinheit. Das Verfahren ist dabei unter einfachsten apparativen bzw. prozesstechnischen Bedingungen sowie bei technisch und ökonomisch sehr gut realisierbaren Temperaturen in nur einer Verfahrensstufe durchzuführen. Das erfindungsgemäße Verfahren wird in Abwesenheit organischer Lösungsmittel und ohne Zusatz weiterer Hilfstoffe oder -komponenten, beispielsweise Kristallisationskeimen, durchgeführt. Es sind keine Derivatisierungen der hinsichtlich des Enantiomerenüberschusses zu reinigenden Ausgangsverbindung notwendig.

Wie eingangs erwähnt, stellt Isopulegol bzw. dessen Isomere wichtige Intermediate zur Herstellung von Menthol bzw. dessen Isomeren dar. Menthol kann durch dem Fachmann an sich bekannte Methoden der Hydrierung, speziell der katalytischen Hydrierung an geeigneten Übergangsmetallkatalysatoren, wie beispielsweise in Pickard et al., J. Chem. Soc. 1920, 1253; Ohloff et al., Chem. Ber. 1962, 95, 1400; Pavia et al., Bull. Soc. Chim. Fr. 1981, 24, Otsuka et al., Synthesis 1991, 665 oder in der EP 1 053 974 A beschrieben, aus Isopulegol erhalten werden. Dabei bleibt bei geeigneter Wahl der Reaktionsbedingungen die relative bzw. absolute Konfiguration des eingesetzten Isopulegols weitgehend, in vielen Fällen vollständig erhalten. Das erfindungsgemäß zugängliche enantiomeren- bzw diastereomerenangereicherte Isopulegol stellt also einen attraktiven Ausgangsstoff zur Herstellung von enantiomeren- bzw. diastereomerenangereichertem Menthol dar.

Die vorliegende Erfindung betrifft demgemäss auch ein Verfahren zur Herstellung von Menthol, umfassend die Schritte
a) Herstellung von enantio- und/oder diastereomerenangereichertem n-Isopulegol der Formel (II) bzw. (II*) durch wie vorstehend beschriebene Kristallisation aus einer n-Isopulegol der Formel (II) bzw. (II*) und gegebenenfalls weitere Diastereomere des Isopulegols enthaltenden Schmelze und
b) Hydrierung des in Schritt a) erhaltenen enantio- und/oder diastereomerenangereicherten n-Isopulegols.

Im Rahmen einer bevorzugten Ausführungsform eignet sich das Verfahren zur Herstellung von L-(-)-Menthol ausgehend von L-(-)-Isopulegol. Letzteres ist auf verschiedenen, dem Fachmann bekannten Wegen zugänglich, beispielsweise durch cyclisierende Oxo-En-Reaktion von optisch aktivem Citronellal in Gegenwart von tris-(2,6-diarylphenoxy)-aluminiumkatalysatoren wie beispielsweise in der EP A-1 225 163 beschrieben.

Dabei ist insbesondere bemerkenswert, dass die Reinigung bzw. Steigerung des Enantiomerenüberschusses von Menthol durch Kristallisation ein bislang technisch nur unzureichend gelöstes Problem darstellt. Angesichts der ausgeprägten chemischen und sterischen Ähnlichkeit von Isopulegol und Menthol ist es als überraschend zu werten, dass die erfindungsgemäße Schmelzekristallisation des Isopulegols nicht mit der gleichen nachteiligen Mischkristallbildung wie im Falle des Menthols erreicht werden kann.

Die folgenden Beispiele dienen der Erläuterung der Erfindung, ohne sie in irgend einer Weise zu beschränken:

### Beispiele:

### Beispiel 1: Statische Schichtkristallisation einer Isopulegol-Schmelze

In einem doppelwandigen Glasrohr als Kristallisator wurden 205 g Isopulegol der Zusammensetzung 95% (-)-n-Isopulegol und 5% (+)-n-Isopulegol (90% ee) mit einem Schmelzpunkt von 13°C bei einer Temperatur 15°C vorgelegt. Der Kristallisator wurde innerhalb von 30 h bis auf 9°C abgekühlt. Das anfangs flüssige Produkt lag am Versuchsende zum großen Teil erstarrt vor. Anschließend wurde die Manteltemperatur innerhalb von 10 h von 13°C auf °C 25°C angehoben. Dabei wurden neben 70 g Mutterlauge und 50 g Schwitzfraktionen 85 g an abgeschmolzener Kristallschicht erhalten. Dieses Endprodukt wies eine optische Reinheit von 99,9% ee bezogen auf (-)-Isopulegol auf.

### Beispiel 2: Dynamische Schichtkristallisation einer Isopulegol-Schmelze

In einem Rührapparat mit planem, über einen Mantel gekühltem Boden (wie in G.F. Arkenbout, Melt Crystallization Technology, Lancater/PA, Technomic Publ. Co., 1995 (Kap. 10.4.1) beschrieben) wurden 1003 g Isopulegol der Zusammensetzung 94,7% (-)-n-Isopulegol und 5,3% (+)-n-Isopulegol (89,4 % ee) mit einem Schmelzpunkt von 10°C bei einer Temperatur von 12°C vorgelegt. Der Kühlmantel des Kristallisatorbodens wurde im Laufe von 2 h auf -14°C abgekühlt. Dabei bildete sich eine 12 mm dicke Kristallschicht mit einem Gewicht von 124 g. Anschließend wurde der Apparat um 180° gedreht und die Manteltemperatur innerhalb von 10 h von 8°C auf 13°C angehoben. Dabei wurden 52 g Schwitzfraktionen und 124 g abgeschmolzene Kristallschicht erhalten. Dieses Endprodukt wies eine optische Reinheit von 99% bezogen auf (-)-n-Isopulegol auf.

### Vergleichsbeispiel 1: Lösungskristallisation von Menthol

In einem 1l-Rührwerkskristallisator wurden 560 g eines Menthol-Isomerengemischs (80% ee, Reinheit bezüglich (-)-Menthol: 90%) in 240 g Aceton gelöst. Die Sättigungstemperatur der Mischung betrug 5,8°C. Nach Abkühlen auf 5,7°C wurde die übersättigte Lösung mit 14 g Impfkristallen reinen (-)-Menthols angeimpft und mit einer Rate von 0,5 bis 1 K/h weiter abgekühlt. Bei Erreichen einer Temperatur von -6,9°C und einem Feststoffgehalt von 22,4 Gew.-% in der Suspension wurde eine Probe gezogen und durch Abzentrifugieren von anhaftender Mutterlösung befreit. Die Kristalle wiesen eine Reinheit von 98,2% (96,4% ee) auf.

### Vergleichsbeispiel 2: Schmelzekristallisation von Menthol

In einem doppelwandigen Glasrohr als Kristallisator wurden 324 g Menthol der Zusammensetzung 95% (-)-Menthol und 5% (+)-Menthol (90% ee) vorgelegt. Der Schmelzpunkt der Mischung lag bei 38°C. Der Kristallisator wurde im Laufe von 15 h von 38,4°C auf 37,4°C abgekühlt. Das anfangs flüssige Produkt lag am Versuchsende fast vollständig erstarrt vor. Anschließend wurde die Manteltemperatur innerhalb von 5 h von 38°C auf 39°C angehoben. Dabei wurden zwei Schwitzfraktionen (51 g und 198 g) sowie 75 g abgeschmolzene Kristallschicht erhalten. Eine Analyse zeigte, dass Ausgangslösung, beide Schwitzfraktionen und die Kristallschicht praktisch identische ee-Werte um 90% aufwiesen.

## Patentansprüche

1. Verfahren zur Herstellung von angereichertem Isopulegol der Formel (I) 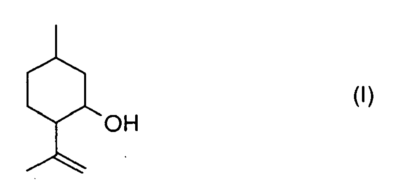 durch Kristallisation aus einer Isopulegol der Formel (I) enthaltenden Schmelze, **dadurch gekennzeichnet, dass** man die Kristallisation aus der Schmelze in Form einer Schichtkristallisation durchführt.

2. Verfahren nach Anspruch 1 zur Herstellung von enantio- und/oder diastereomerenangereichertem n-Isopulegol der Formel (II) 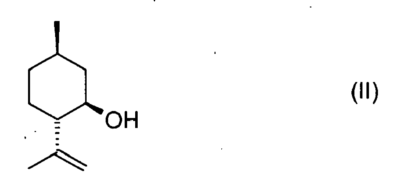 durch Kristallisation aus einer n-Isopulegol der Formel (II) und gegebenenfalls weitere Diastereomere des Isopulegols enthaltenden Schmelze.

3. Verfahren nach Anspruch 1 oder 2, **dadurch gekennzeichnet, dass** die Schmelze zu mindestens 70 Gew.-% aus Isopulegol der Formel (I) besteht.

4. Verfahren nach einem der Ansprüche 1 bis 3, **dadurch gekennzeichnet, dass** die Schmelze zu mindestens 70 Gew.-% aus n-Isopulegol der Formel (II) besteht.

5. Verfahren nach einem der Ansprüche 1 bis 4 zur Herstellung von enantio- und diastereomerenangereichertem n-Isopulegol durch Kristallisation aus einer optisch aktives n-Isopulegol mit geringerem Enantiomerenüberschuss enthaltenden Schmelze.

6. Verfahren nach einem der Ansprüche 1 bis 5, **dadurch gekennzeichnet, dass** man die Kristallisation aus der Schmelze bei Temperaturen im Bereich von-20°C bis 15°C durchführt.

7. Verfahren nach einem der Ansprüche 1 bis 6, **dadurch gekennzeichnet, dass** man die Kristallisation statisch oder dynamisch durchführt.

8. Verfahren nach einem der Ansprüche 1 bis 7, **dadurch gekennzeichnet, dass** man die Kristallisation aus der Schmelze in Abwesenheit von Kristallisationskeimen durchführt.

9. Verfahren nach einem der Ansprüche 2 bis 8, **dadurch gekennzeichnet, dass** man n-Isopulegol mit einem Enantiomerenüberschuss von mindestens 75 % ee einsetzt.

10. Verfahren nach einem der Ansprüche 1 bis 9 zur Herstellung von Isopulegol mit einer chemischen Reinheit von mindestens 95%.

11. Verfahren nach einem der Ansprüche 2 bis 10 zur Herstellung von n-Isopulegol mit einem Enatiomerenüberschuss von mindestens 85% ee.

12. Verfahren nach einem der Ansprüche 2 bis 11, zur Herstellung von enantiomerenangereichertem L-(-)-n-Isopulegol.

13. Verfahren zur Herstellung von Menthol, umfassend die Schritte
a) Herstellung von enantio- und/oder diastereomerenangereichertem n-Isopulegol der Formel (II) durch Kristallisation aus einer n-Isopulegol der Formel (II) und gegebenenfalls weitere Diastereomere des Isopulegols enthaltenden Schmelze nach einem der Ansprüche 2 bis 12 und
b) Hydrierung des in Schritt a) erhaltenen enantio- und/oder diastereomerenangereicherten n-Isopulegols.

14. Verfahren nach Anspruch 13 zur Herstellung von L-(-)-Menthol umfassend die Kristallisation von L-(-)-n-Isopulegol enthaltenden Schmelze.

## Claims

1. A process for preparing enriched isopulegol of the formula (I) 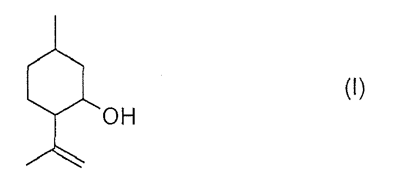 by crystallization from a melt comprising isopulegol of the formula (I), which comprises performing the crystallization from the melt in the form of a layer crystallization.

2. The process according to claim 1 for preparing enantiomerically and/or diastereomerically enriched n-isopulegol of the formula (II) 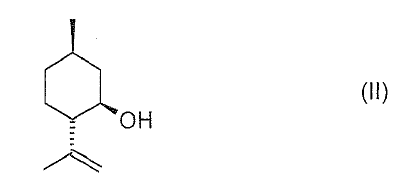 by crystallization from a melt comprising n-isopulegol of the formula (II), and possibly further diastereomers of isopulegol.

3. The process according to claim 1 or 2, wherein the melt consists of isopulegol of the formula (I) to an extent of at least 70% by weight.

4. The process according to any of claims 1 to 3, wherein the melt consists of n-isopulegol of the formula (II) to an extent of at least 70% by weight.

5. The process according to any of claims 1 to 4 for preparing enantiomerically and diastereomerically enriched n-isopulegol by crystallization from a melt comprising optically active n-isopulegol with a lower enantiomeric excess.

6. The process according to any of claims 1 to 5, wherein the crystallization from the melt is performed at temperatures in the range from -20°C to 15°C.

7. The process according to claim 1 or 6, wherein the crystallization is performed in a static or dynamic manner.

8. The process according to any of claims 1 to 7, wherein the crystallization from the melt is performed in the absence of crystallization seeds.

9. The process according to any of claims 2 to 8, wherein n-isopulegol having an enantiomeric excess of at least 75% ee is used.

10. The process according to any of claims 1 to 9 for preparing isopulegol having a chemical purity of at least 95%.

11. The process according to any of claims 2 to 10 for preparing n-isopulegol having an enantiomeric excess of at least 85% ee.

12. The process according to any of claims 2 to 11 for preparing enantiomerically enriched L-(-)-n-isopulegol.

13. A process for preparing menthol, comprising the steps of
a) preparing enantiomerically and/or diastereomerically enriched n-isopulegol of the formula (II) by crystallization from a melt comprising n-isopulegol of the formula (II) and possibly further diastereomers of isopulegol according to any of claims 2 to 14 and
b) hydrogenating the enantiomerically and/or diastereomerically enriched n-isopulegol obtained in step a).

14. The process according to claim 13 for preparing L-(-)-menthol, comprising the crystallization of melt comprising L-(-)-n-isopulegol.

## Revendications

1. Procédé pour la préparation d'isopulegol enrichi de formule (I) 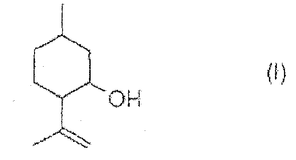 par cristallisation d'une masse fondue contenant de l'isopulegol de formule (I), **caractérisé en ce qu'**on réalise la cristallisation à partir de la masse fondue sous forme d'une cristallisation en couches.

2. Procédé selon la revendication 1 pour la préparation de n-isopulegol enrichi en énantiomères et/ou diastéréoisomères de formule (II) 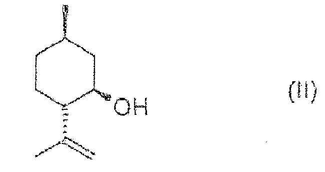 par cristallisation à partir d'une masse fondue contenant du n-isopulegol de formule (II) et le cas échéant d'autres diastéréoisomères de l'isopulegol.

3. Procédé selon la revendication 1 ou 2, **caractérisé en ce que** la masse fondue est constituée à raison d'au moins 70% en poids d'isopulegol de formule (I).

4. Procédé selon l'une quelconque des revendications 1 à 3, **caractérisé en ce que** la masse fondue est constituée à raison d'au moins 70% en poids de n-isopulegol de formule (II).

5. Procédé selon l'une quelconque des revendications 1 à 4 pour la préparation de n-isopulegol enrichi en énantiomères et diastéréoisomères par cristallisation à partir d'une masse fondue contenant du n-isopulegol optiquement actif avec un faible excès d'énantiomères.

6. Procédé selon l'une quelconque des revendications 1 à 5, **caractérisé en ce qu'**on réalise la cristallisation à partir de la masse fondue à des températures dans la plage de -20 à 15°C.

7. Procédé selon l'une quelconque des revendications 1 à 6, **caractérisé en ce qu'**on réalise la cristallisation statiquement ou dynamiquement.

8. Procédé selon l'une quelconque des revendications 1 à 7, **caractérisé en ce qu'**on réalise la cristallisation à partir de la masse fondue en l'absence de germes de cristallisation.

9. Procédé selon l'une quelconque des revendications 2 à 8, **caractérisé en ce qu'**on utilise du n-isopulegol avec un excès d'énantiomères d'au moins 75% d'ee.

10. Procédé selon l'une quelconque des revendications 1 à 9 pour la préparation d'isopulegol présentant une pureté chimique d'au moins 95%.

11. Procédé selon l'une quelconque des revendications 2 à 10 pour la préparation de n-isopulegol avec un excès d'énantiomères d'au moins 85% d'ee.

12. Procédé selon l'une quelconque des revendications 2 à 11 pour la préparation de L-(-)-n-isopulegol enrichi en énantiomères.

13. Procédé pour la préparation de menthol, comprenant les étapes
a) préparation de n-isopulegol enrichi en énantiomères et/ou en diastéréoisomères de formule (II) par cristallisation à partir d'une masse fondue contenant du n-isopulegol de formule (II) et le cas échéant d'autres diastéréoisomères de l'isopulegol selon l'une quelconque des revendications 2 à 12 et
b) hydrogénation du n-isopulegol enrichi en énantiomères et/ou en diastéréoisomères obtenu dans l'étape a).

14. Procédé selon la revendication 13 pour la préparation de L-(-)-menthol comprenant la cristallisation d'une masse fondue contenant du L-(-)-n-isopulegol.
